(19) Europäisches Patentamt
European Patent Office
Office européen des brevets

(11) Publication number: 0 143 564
B1

(12) **EUROPEAN PATENT SPECIFICATION**

(45) Date of publication of patent specification: **14.06.89**

(51) Int. Cl.$^4$: **C 07 D 323/00**, G 01 N 27/30, B 01 D 13/04

(21) Application number: **84307540.9**

(22) Date of filing: **01.11.84**

(54) **Crown ether Derivatives.**

(30) Priority: **02.11.83 HU 199981**

(43) Date of publication of application: **05.06.85 Bulletin 85/23**

(45) Publication of the grant of the patent: **14.06.89 Bulletin 89/24**

(84) Designated Contracting States: **AT BE CH DE FR GB IT LI NL SE**

(56) References cited:
**WO-A-83/00149**
**US-A-4 361 473**

**BULLETIN OF THE CHEMICAL SOCIETY OF JAPAN, vol. 53, February 1980, pages 547-548, The Chemical Society of Japan, Tokyo, JP; H. TAMURA et al.: "Effect of plasticizer on the selectivity of potassium-selective PVC membrane electrodes based on bis(crown ethers)"**

**CHEMICAL ABSTRACTS, vol. 98, 1983, page 510, no. 16661a, Columbus, Ohio, US; A. BELA et al.: "Modified crown ethers. II. Carbonic acid derivatives of bis-crown ethers", ACTA CHIM. ACAD. SCI. HUNG. 1982, 110(1), 29-33**

(73) Proprietor: **MAGYAR TUDOMANYOS AKADEMIA KUTATAS-ES SZERVEZETELEMZÖ INTEZET**
**18 Münnich Ferenc u.**
**H-1051 Budapest (HU)**

(72) Inventor: **Tóke, Lászlo**
**14 Hajnóczy u.**
**Budapest XII (HU)**
Inventor: **Agai, Béla**
**5 Miklos u.**
**Budapest III (HU)**
Inventor: **Bitter, István**
**76 Ady E. u.**
**Budapest XIX (HU)**
Inventor: **Pungor, Ernó**
**7 Meredek u.**
**Budapest XI (HU)**
Inventor: **Szepesváry, Klára**
**46 Nánasi u.**
**Budapest III (HU)**
Inventor: **Lindner, Ernó**
**6 Kiscelli köz**
**Budapest III (HU)**
Inventor: **Forró, Mária**
**78 Váci ut**
**Budapest XIII (HU)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

EP 0 143 564 B1

Courier Press, Leamington Spa, England.

(56) References cited:

CHEMICAL ABSTRACTS, vol. 98, 1983, page 509, no. 16660z, Columbus, Ohio, US; A. BELA et al.: "Modified crown ethers. I. Carbonic acid derivatives containing one crown ether unit", ACTA CHIM. ACAD. SCI. HUNG. 1982, 110(1), 25-8

(72) Inventor: Havas, Jenó
32 Remetehegy ut
Budapest III (HU)

(74) Representative: Pett, Christopher Phineas et al
Frank B. Dehn & Co. European Patent Attorneys
Imperial House 15-19 Kingsway
London WC2B 6UZ (GB)

**Description**

This invention relates to new crown ether derivatives, to processes for the preparation thereof and to ion-selective membrane electrodes comprising the same. More particularly the invention is concerned with new crown ether derivatives useful as cation complexing agents.

The new crown ether derivatives of the present invention can be used for cation complex formation under more favourable conditions and provide better results than the known ethers belonging to the prior art; the lifetime of membrane sensors comprising the new crown ether derivatives of the present invention used in ion-selective electrodes is longer than that of known membranes and certain analytical characteristic data are better as well.

The invention also relates to ion-selective membrane electrodes comprising a new crown ether derivative of the present invention, the said membranes having a longer lifetime than those prepared from known crown compounds while the performance of the membrane electrodes of the present invention is at least as high as that of the known products.

In the field of biochemistry, the measurement of the potassium ion is often of particular importance. A considerable amount of research has therefore concentrated on developing potassium selective electrodes which give better and more consistent results.

Swiss patent No. 479,870 describes an ion-selective membrane electrode prepared using valinomycin as active substance but although the selectivity and potential response of such electrodes are generally favourable, the $Rb^+$ and $Cs^+$ ion-selectivity are not satisfactory for certain fields of application.

In our International Patent Publication No. WO 83/00149 we describe, *inter alia* compounds of the general formula (V)

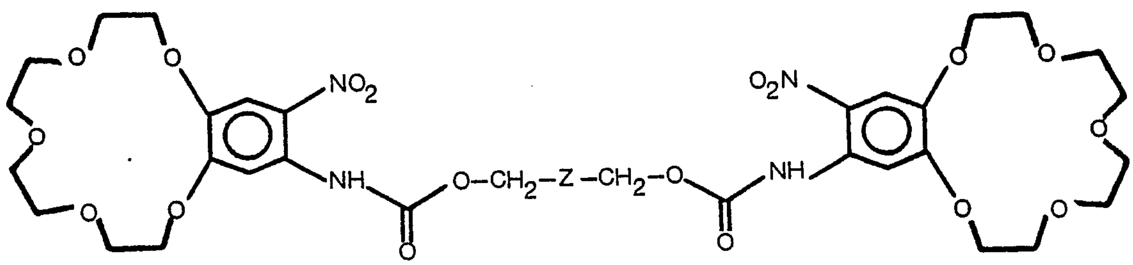

(wherein Z represents a chemical bond, $-CH_2-$, $-(CH_2)-_{2-4}$, $-CH_2OCH_2-$, $-CH_2SCH_2-$ or a cyclic group) which compounds, when used as ion-selective membrane ligands exhibit a high $K^+$ ion-selectivity thus permitting good analytical determination of $K^+$ ion concentration in the presence of other interferring alkali metal or alkaline earth metal ions such as $Na^+$, $Ca^{2+}$, $Mg^{2+}$, $Rb^+$ or $Cs^+$. The main analytical characteristics of electrodes prepared using these active substances (e.g. selectivity, potential response) are equivalent to those achieved using valinomycin while the $Rb^+$ and $Cs^+$ ion selectivity are significantly better.

It is known [Kimura *et al*: J. Electroanal. Chem. *95,* 91—101 (1979)] that certain compounds belonging to the class of bis-benzo-15-crown-5-derivatives shown $K^+$ ion selectivity. These compounds are not, however, widely used in ion selective electrodes since (as is described in Microchimica Acta II. 287—296, 1983) the lifetime of membrane electrodes prepared using these compounds is not satisfactory and the electrodes lose their selectivity after about 200 measurements.

It was thought that the good ion selectivity of the compounds disclosed in our International Patent Publication No. WO 83/00149 was due to the fact that although the nitro group attached to the benzene ring of the benzo crown ether decreases the complex stability coefficient of the metal ion (this does not favour selectivity per se), the intramolecular hydrogen bridge which may be formed with the adjacent —NH group forces the two crown ether rings remote from each other into a favourable stereoconformation and results in the compounds of general formula V, in an overall increase in $K^+$ stability. This, it was thought, a $K^+$ ion trap is formed having internal dimensions which make it capable only of complexing $K^+$ ions because the larger ions ($Rb^+$, $Cs^+$) cannot "fit in" while the smaller ions ($Na^+$, $Li^+$) "fall out". The potassium ion on the other hand, forms a "sandwich".

Based on these ideas we were previously of the opinion that the "selectivity" would not depend on the shape and length of the chain connecting the crown ether units".

Membrane electrodes incorporating compounds of general formula (V) above have been used to determine the potassium content of natural and industrial waters. On using such electrodes for repeated or continuous measurements in biological systems it has been found, however, that the lifetime of the membrane is not always satisfactory, presumably due to the slow dissolution of the insufficiently lipophilic ligands. Thus the lifetime of the membrane may not be sufficient for continuous *in vivo* measurements such as the continuous control of the condition of a patient during surgical operations or in long-lasting automatic series of measurements.

It would thus be desirable to provide new crown compounds which ensure a longer lifetime for the ion-selective membrane electrode while maintaining the other favourable properties at least at their original

level and if possible even improving them.

The present invention is based on our finding that, while maintaining the possibility of hydrogen bridge formation as described above, the lifetime can be increased and the $K^+$ ion selectivity even increased to a small extent, by replacing the Z group forming the pole of symmetry of the symmetrical chain linking the two benzo crown ether parts in the compounds of general formula V above by a group of the Formula $Z^1$:

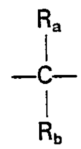

(wherein $R_a$ represents an alkyl group having 1—4 carbon atoms and $R_b$ represents an alkyl group having 1—20 carbon atoms).

Thus according to one feature of the present invention we provide crown compounds of general formula I

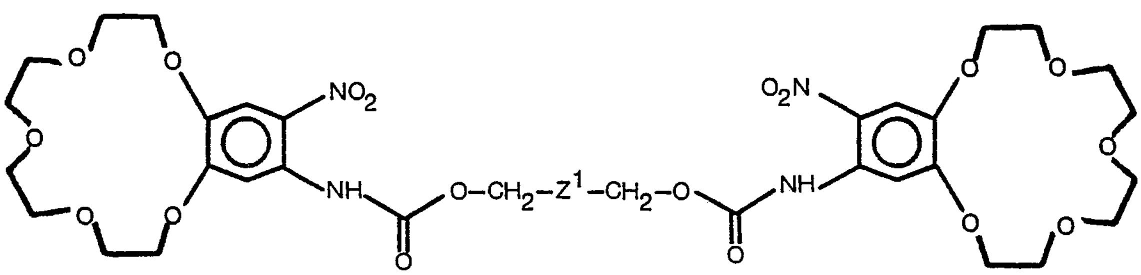
(I)

wherein

$Z^1$ is a group of formula

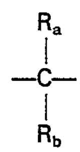

in which $R_a$ represents an alkyl group having 1—4 carbon atoms and $R_b$ represents an alkyl group having 1—20 carbon atoms.

The structural difference between the crown ether derivatives of the present invention and those disclosed in International Patent Publication WO 83/00149 clearly resides in the difference between the groups Z and $Z^1$. The effects resulting from this structural modification are however surprising and very significant.

Whilst it might have been thought obvious to eliminate the possibility of hydrogen bridge formation in order to increase the lipophilicity in an advantageous manner, this cannot be done because e.g. methylation of the NH moiety of the carbamate group strongly reduces selectivity and the slope of the calibration curve of the electrode prepared from such N-methyl derivatives falls below that of the theoretical 59.16 mV/decade value.

Replacement of the group Z in the compounds of general formula V by group $Z^1$ in the pole of symmetry of the bridging chain (i.e. in position 2 of the propylene group which constitutes the central part) surprisingly not only lengthens the lifetime, but the $K^+$ selectivity is also improved to a certain extent. The following additional effects and advantages are also achieved: since the hydrogen bridge is maintained the slope of the calibration curve approaches the theoretical value; practically no "drift" is observed during measurement; the reproducibility of the measurements improves significantly; the sensitivity to stirring is decreased.

According to a further feature of the present invention there are provided potassium ion-selective membrane electrodes comprising, as active substance at least one crown ether compound of general formula I as defined above.

The compounds of the general formula I differ from those of the general formula V by the presence of group $Z^1$ in position 2 of the propylene group in the place of group Z. Both, however, are capable of hydrogen bridge formation as discussed above and the role of the hydrogen bridge in achieving good selectivity is underlined by the fact that if the nitro group is removed, the compounds of the general formula I' thus obtained possess significantly less selective properties

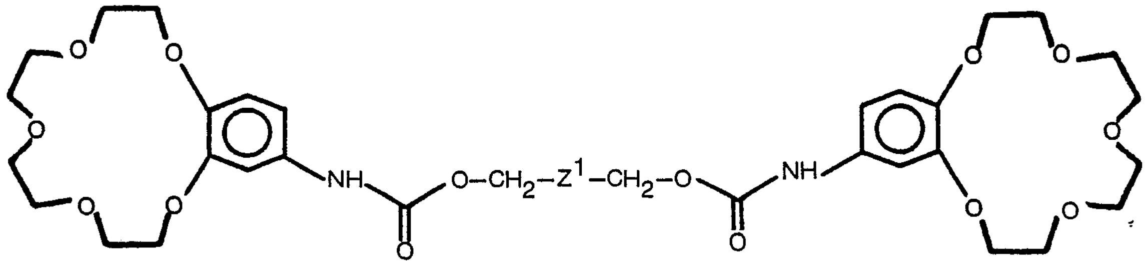

(I')

In order to assess the properties of the compounds of the present invention, reference is made herein to compound of general formula VI

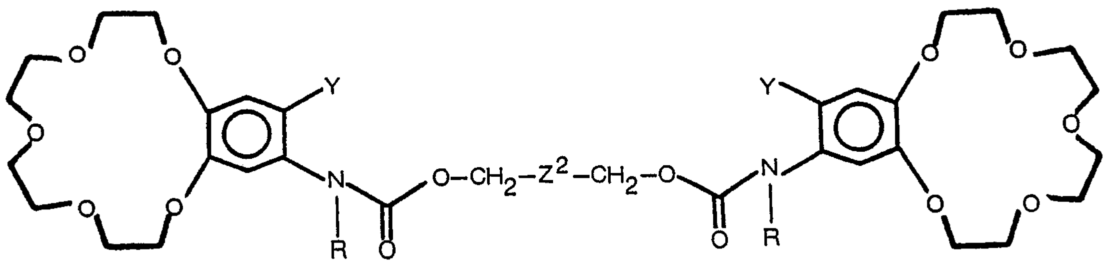

(VI)

(wherein Y is hydrogen or nitro, R is hydrogen or methyl and $Z^2$ stands for Z or $Z^1$) which formula includes *inter alia* compounds of the general formulae I, I' and V.

Substituent $R_a$ attached to the carbon atom of group $Z^1$ is preferably methyl, ethyl, *n*-propyl or *n*-butyl particularly methyl, ethyl or *n*-butyl. $R_b$ stands preferably for ethyl, *n*-butyl, *n*-octyl, *n*-dodecyl or *n*-hexadecyl. The chain length of substituent $R_a$ and $R_b$ may be identical or different. If $R_a$ and $R_b$ are of identical chain length then they may or may not be identical.

The compounds of the general formula I may be prepared either by total synthesis or by nitrating a crown compound of the general formula I' containing no nitro group. Such processes constitute further features of the present invention.

According to the total synthesis a crown compound of the formula II

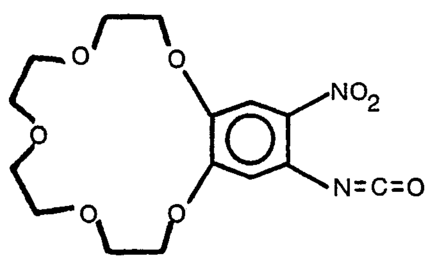

(II)

is reacted with a compound of formula III

$$HO—CH_2—Z^1—CH_2—OH \qquad (III)$$

(wherein $Z^1$ is as defined above) in an aprotic solvent.

The nitration is carried out by reacting a crown compound of formula I' with nitric acid.

The total synthesis may preferably be carried out by reacting the compounds of the general formulae II and III in a molar ratio of 2:1 at a temperature of between 0°C and 50°C in a chlorinated hydrocarbon or a solvent of the ether type, optionally in the presence of a tertiary amine (e.g. triethylamine) as catalyst. The product may be isolated by removing the solvent and purified by recrystallization from a suitable organic solvent (e.g. ethyl acetate or methyl isobutyl ketone).

Nitration of a compound of formula I' with nitric acid may preferably be carried out in acetic acid or a chlorinated hydrocarbon as solvent at a temperature ranging from 30°C to 70°C. The product may be isolated by cooling, filtration or pouring on ice and subsequently by extracting with a chlorinated hydrocarbon. The product may be purified by recrystallization from the above solvent mixture.

The starting material of formula II is described in International Patent Publication No. WO 83/00149. The isocyanate may be obtained by reacting an amine of the formula IV

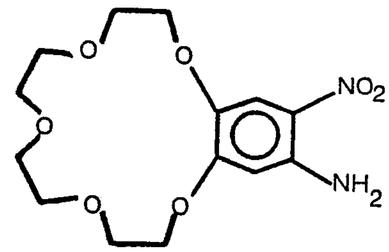

(IV)

with phosgene in an aromatic solvent having a high boiling point in a manner known per se.

The compounds of general formula I or mixtures thereof can be used as active substance in ion-selective electrodes in conventional manner e.g. as follows:

a) The selected compound(s) is/are built into a carrier to form the membrane e.g. using a PVC carrier phase, generally in an amount of 0.1—10%, preferably 0.2—10% of the weight of the membrane. Optionally a suitable plasticizer is used, preferably a phthalic acid ester, e.g. dipentyl phthalate (DPP), a sebacic acid ester e.g. dioctyl sebacate (DOS) or *ortho*-nitro-phenyl-octyl ether (ONPOE) or a mixture thereof. The plasticizer may for example be present in an amount of 55—75% by weight of the membrane.

b) The selected compound(s) is/are built in an amount of 0.2—10% of the weight of the membrane into a suitable polymer — preferably silicon rubber or PVC, polyamide, or polyethylene — which has a dielectric constant in the range between 2 and 30.

c) Applying a solution of the selection compound(s) in a suitable solvent (preferably in a plasticizer as described in paragraph a above) onto a porous membrane.

The membranes prepared as described above may be installed into a suitable electrode and connected together with a suitable reference electrode to provide an analytical instrument.

The correlation between cell voltage and logarithm activity ($-\log a_{K^+}=pK$) is linear in the range of pK=1—5.0 and can be characterized by the so-called modified Nikolsky equation:

$$EME = E_o + S_{log}\,(a_i + \sum_{i \neq j} K^{pot}\, a_j^{\,z_i/z_j}) + E_D$$

wherein

EME = cell voltage

$E_o$ = constant reference potential independent of the sample/solution

$E_D$ = sum of the diffusion potentials in the cell

$a_i, a_j$ = activity of the ion to be measured and of the interfering ions

$z_i, z_j$ = number of charge of the ion to be measured and of the interfering ions

S = so called Nernst factor, the value of which amounts to 59.16 mV/$z_i$ at 25°C

$K_{ij}^{pot}$ = selectivity factor.

The present invention is illustrated by the following non-limiting Examples.

## Examples

Various compounds are referred to in these Examples by reference to their serial numbers as denoted in Table 1 which follows. Beside the serial number, the "internal number" of the new compounds of the present invention and the compounds disclosed in our International Patent Publication WO 83/00149 are also indicated, the "internal number" comprising the letters BME and a two-figure number. The compounds have been divided into groups A, B and C, the compounds of the present invention constituting group A, the compounds disclosed in the International Patent Publication WO 83/00149 constituting group B and other reference compounds constituting group C.

TABLE 1

| Group | Ser. No. | Internal No. | General Formula VI | | |
|---|---|---|---|---|---|
| | | | Y | $Z^2$ | R |
| "A" | 1 | BME-44 | $NO_2$ | 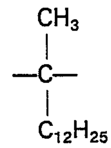 | H |
| compounds of present invention | 2 | BME-53 | $NO_2$ | 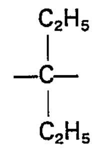 | H |
| | 3 | BME-54 | $NO_2$ | 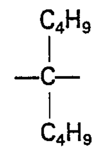 | H |
| "B" compound of WO 83/00149 | 4 | BME-36 | H | $CH_2SCH_2$ | H |
| | 5 | BME-15 | $NO_2$ | $CH_2SCH_2$ | H |
| | 6 | BME-15-Me | $NO_2$ | $CH_2SCH_2$ | $CH_2$ |
| | 7 | BME-36/2 | H | $CH_2CH_2CH_2$ | H |
| | 8 | BME-71 | $NO_2$ | $CH_2CH_2CH_2$ | H |
| "C" referential compounds | 9 | $R_V$: Valinomycin | | | |
| | 10 | $R_K$: Kimura et al: J. Electroanal. Chem. *95*/1979/page 92 III. /n=3/ crown ether derivatives | | | |

The following reference compounds are used:

$R_V$ = Valinomycin (in a Philips IS 561-$K^+$ electrode)

$R_K$ = Crown ether derivatives prepared according to Kimura et al: J. Electroanal. Chem. *95* (1979) 91—101.

Preparation of bis crown compounds of the general formula I

a) Total synthesis

To a solution of 7.08 g (20 millimoles) of the isocyanate of the formula II in 60 ml of anhydrous chloroform 1.32 g (10 millimoles) of 2,2-diethylpropane-1,3-diol

III:

$Z^1$ = 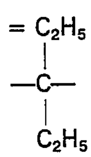

are added. After complete dissolution 2 drops of triethylamine are added to the solution and the reaction mixture is stirred at room temperature for 2 hours. The solvent is distilled off *in vacuo*. The yellow solid residue is triturated with ether. The crystalline product is filtered and dried at room temperature. Thus 7.73 g (92%) of the crude product 2. are obtained (BME-53, see Table 1). The product is recrystallized from a mixture of ethyl acetate and ether. Thus 5.76 g of the pure product are obtained (68.5%). M.p.: 118—119°C.

The reaction parameters used in analogous preparations of further compounds of the general formula I are disclosed in Table 2. The physical, analytical and spectroscopical data of the products are enumerated in Table 3.

TABLE 2

Reaction conditions of preparation of bis crown compounds of the general Formula I

| Ser. No. (Int. No.) | Solvent /catalyst/ | Reaction time/hour/ Temperature/°C/ | Yield % |
|---|---|---|---|
| 1 | $CHCl_3$ | 8 | |
| /BME-44/ | %-/ | /30/ | 55 |
| | $CHCl_3$ | | |
| | /TEA/ | 3/30/ | 63 |
| | Dioxan | 1/50/ | 61.5 |
| 2 | $CHCl_3$ | 8/25/ | 64 |
| /BME-53/ | /-/ | | |
| | $CHCl_3$ | 3/25/ | 68.5 |
| 3 | Dioxan | 6/30/ | 61 |
| /BMME-54/ | /-/ Dioxan /TEA/ | 1/50/ | 66 |

TEA: triethyl amine

TABLE 3

Physical constants of compounds of the general Formula I

| Ser. No. (Int. No.) | $Z^1$ | Mp. °C | Empirical formula /M.w./ | Analysis /%/ calculated | found | IR/KBr/ $cm^{-1}$ vc=OvNH |
|---|---|---|---|---|---|---|
| 1 /BME-44/ | 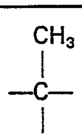 | 76—77 | $C_{46}H_{70}N_4O_{18}$ /966/ | C 57.14<br>H 7.24<br>N 5.79 | C 57.05<br>H 7.18<br>N 5.69 | 1720 3310 |
| 2 /BME-53/ | 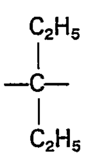 | 117—119 | $C_{37}H_{52}N_4O_{18}$ 840 | C 52.65<br>H 6.18<br>N 6.67 | C 52.48<br>H 6.11<br>N 6.60 | 1715 3310 |
| 3 /BME-54/ | 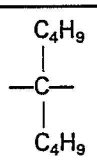 | 95—96 | $C_{41}H_{60}N_4O_{18}$ 896 | C 54.91<br>H 6.69<br>N 6.25 | C 54.85<br>H 6.60<br>N 6.17 | 1720 3310 |

b) Nitration

To a solution of 6.26 g (20 millimole) N-3,4-(1′,4′,7′,10′,13′-pentaoxacyclopentadeca-2-ene)-phenyl isocyanate in 60 ml of anhydrous chloroform 1.32 g (10 millimole) of 2,2-diethyl-propane-1,3-diol are added.

III: $Z^1$ = 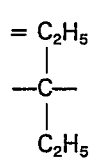

The reaction mixture is stirred at 50°C for 5 hours and cooled to 20°C. To the reaction mixture a solution of 3 ml of 65% (specific weight 1.42) nitric acid and 10 ml of glacial acetic acid are added dropwise. The mixture is stirred at room-temperature for a further 10 minutes and at 65°C for 30 minutes. The reaction mixture is cooled, poured onto crushed ice and the two phases are separated. The aqueous layer is extracted four times with 20 ml of chloroform each. The united organic phases are washed neutral with cold water, dried over magnesium sulfate, filtered and the chloroform solution is evaporated. The residue is purified according to method a).

Yield: 6.23 g (72.5%). Product 2 (BME-53).

In an analoogus manner compounds 1 (BME-44, yield 57%) and 3 (BME-54, yield 61%) are prepared.

Preparation of a potassium ion-selective membrane using crown compounds of general formula I

1) 30—40, preferably about 34 parts by weight of PVC powder (e.g. SDP Hochmolekular, from Lonza AG., Basel, Switzerland) are dissolved in about 2—3 ml of tetrahydrofuran, and this solution is poured into a vessel containing 0.1—10 parts by weight, preferably 2 parts by weight of active ingredient (ionophor) and 65—75, preferably 63.5 parts by weight of plasticizer (an organic solvent of a dielectrical constant of 2—30, and 0—5, preferably 0.5 part by weight of an additive (e.g. sodium-tetraphenyl-borate or potassium p-chloro-tetraphenyl-borate).

On a glass plate of flat surface a glass ring of a height of 10 mm and diameter of 25—35 mm is fixed by means of a rubber ring whereafter about 3 ml of the prepared mixture is poured on the glass ring and covered with a filter paper. Tetrahydrofuran evaporates at room temperature within 1—2 days through the filter paper and at the bottom of the glass ring an elastic membrane of 0.1—0.5 mm thickness remains as a residue which can be collected readily from the glass plate.

2) 0.2—10, preferably 2.5 parts by weight of active ingredient and 80—100 mg preferably 83 parts by weight of dimethyl polysiloxane (e.g. Silopren K 18000, Farbenfabrik Bayer) are dissolved in about 2—4 ml of carbon tetrachloride. To the solution a crosslinking agent in appropriate amount is added (preferably 14.5 parts by weight). Any cold curing catalyst suitable for cross-linking, such as T-37, (Wacker Chemie GmbH, München) can be used and the mixture is then poured into the glass ring fixed on the glass plate described in 1 and one may further proceed as disclosed in 1.

3) 0—10, preferably 2.5 parts by weight of active ingredient are pulverized in an achate mortar and dispersed in 80—100 mg preferably 83 parts by weight of dimethylpolysiloxane (e.g. Silapren K-1000, Farbenfabrik Bayer) until a statistically homogeneous suspension is obtained. Crosslinking agent is added in an amount necessary for curing (e.g. 1 part by weight of dibutyl-Sn-dilaurate and 2 parts by weight of hexaethoxy siloxane) whereafter the mixture is applied by a plastic plate at an equal extent in a thickness of 0.01—1 mm. After curing, which takes at most a few hours, the elastic plate can be collected from the plastic plate.

4) 0.1—20 parts by weight of active ingredient are dissolved in water-immiscible solvent (dielectric constant of which is 2—30) such as phthalic acid ester, dipentyl phthalate, sebacic acid esters, (dioctyl sebacate), *o*-nitrophenyl-octyl ether and a mixture thereof, and a porous membrane is wet with the thus obtained solution.

5) The tetrahydrofuran solution prepared according to 1 containing plasticizer and active ingredient or a carbon tetrachloride dimethylpolysiloxane solution prepared according to 2 containing active ingredient and catalyst or a dimethylpolysiloxane active ingredient suspension prepared according to 3 containing crosslinking agent and/or a catalyst is applied to an electron-conducting substance, preferably metal silver, silver chloride wire, platinum or gold fibre, graphite rod etc.

*Electrochemical properties of some selected active substances of the general formula I used in potassium ion-selective membrane electrodes*

A PVC or silicon rubber carrier-phase membrane was prepared from the selected active substances (e.g. Nos. 1, 2 or 3) according to Examples 1 and 2, whereafter it was built into a suitable electrode body (such as a Philips IS-561 Liquid membrane electrode body) and the following measuring cell was formed:

Ag, AgCl | 0.01 M KCl | ion-selective membrane | sample solution

ion-selective electrode

| 0.1 M $LiOOCCH_3$ | 0.1 M KCl | Ag, AgCL

double junction reference electrode

EME — log $a_{K^+}$ calibration curves were plotted in the range of $10^{-1}$M KCl-$10^{-6}$ M KCl using the potential data measured with the above measuring cell. The selectivity factors of the electrodes were determined by the so-called separate solution method (Moody, G. J., Thomas, J. D. R.: Selective Ion-Sensitive Electrodes, Watford, Merrow Publishing Co. Ltd., England 1971) by using solutions of concentration of $10^{-1}$ M.

For the sake of simpler comparability the calibration data of electrodes comprising as active substance a compound of the general formula I is given in Figure 1 and Table 4 and the selectivity factors relating to the different ions is given in Figure 2 and Table 5) together with the corresponding data of electrodes

prepared using the compounds of the present invention and reference compounds. On comparing the calibration and selectivity data of electrodes prepared by using compounds Ser. Nos. 1, 2 and 3 by means of the same membrane and electrode manufacturing process, respectively, it has been found — contrary to earlier thought — that the modification of the bridging chain while maintaining the —$NO_2$ and —NH groups results in a significant increase in K-concentration sensitivity (S approx. theoretical) and potassium-ion selectivity considerably improves in the presence of sodium-ions ($-\log K^{pot}_{K,Na} = 3.5$). This is of particular importance if the potassium content of biological samples is followed or measured, respectively.

It is of primary importance in use in automatic analysing devices with streaming solutions that the sign measured with the ion-selective electrodes should not at all be dependent on the streaming velocity and stirring rate of the sample solution or should be dependent, but to a small extent, in accordance with theoretical considerations. The dependence of the sign measured by potassium ion-selective electrodes prepared using BME-15, BME-44 and valinomycin ligands, respectively, on the rate of stirring is shown in Figure 3. The values are measured in a potassium chloride solution having a concentration of $10^{-1}$ mole/l by using a laboratory magnetic stirrer. It can be seen from the data of the said Figure that the stirring-sensitivity of the ion-selective electrode comprising BME-44 is much smaller than that of the electrode comprising BME-15 and is approximately the same as that comprising valinomycin.

From the point of view of highly accurate measurement of the $K^+$ content in biological samples a particularly important data is the reproducibility of the potential measurement which amounts to $\pm$ 0.1 mV in the case of ion-selective electrodes containing BME-44 ligand both in an aqueous potassium chloride solution corresponding to the composition of blood serum and also in blood serum, respectively.

The lifetime of ion-sensitive electrodes is a very important characteristic feature and this parameter is determined by measuring the calibration and selectivity data of the electrodes at various points in time. Between the measurements the electrodes are stored in a large volume (300—400 ml) of distilled water. It has been found that during a period of one year the potassium concentration sensitivity (S value) of electrodes comprising the product BME-44 decreases by 1—2 mV/decade, while the selectivity data change but to an insignificant extent. During this period the $E_o$ value show a standard deviation of but a few mV.

The potassium-ion sensitivity of ion-sensitive electrodes before storage in blood serum and a month after storage, respectively is shown in Table 6. It appears from similar experiments that the ion-sensitive electrode prepared from compound BME-44 is suitable for the determination of $K^+$ ions in blood serum for more than three months.

On comparing the characteristic data of potassium-ion sensitive electrodes prepared by using various ionophores in the same manner it can be stated unambiguously that if the benzo crown ether units are bridged by a branched chain the parameter improve in all cases.

In the specification the following compounds are used:

Compound 1 (BME-44): 2 - n - dodecyl - 2 - methyl - propylene - 1,3 - bis - N,N' - [2'' - nitro - 4'',5''-(1''',4''',7''',10''',13'''-pentaoxa-cyclopentadeca-2'''-ene)-phenyl]-carbamate;

Compound 2 (BME-53): 2,2-diethyl-propylene-1,3-bis-N,N'-[2''-nitro-4'',5''-(1''',4''',7''',10''',13'''-pentaoxa-cyclopentadeca-2'''-ene)-phenyl]-carbamate;

Compound 3 (BME-54): 2,2-di-n-butyl-propylene-1,3-bis-N,N'-[2''-nitro-4'',5''-(1''',4''',7''',10''',13'''-pentaoxa-cyclopentadeca-2'''-ene)-phenyl]-carbamate.

The following abbreviations are used throughout the specification:

ONPOE = *o*-nitro-phenyl octyl ether
NaTPB = sodium tetraphenyl borate
SR = silicon rubber
DPP = dipentyl phthalate
DOS = dioctyl sebacate

In Table 4 which follows the electrochemical data is summarised for comparative purposes.

Table 4

Analytical constants of potassium ion selective electrodes

| No. | Membrane Matrix | Additive | Slope (mV) decade | Concentration interval ($-\log a_i$) | Logarithm of selectivity coefficient ($\log K^{pot}$)* j: Na | $NH_4$ | Rb | Cs | Li | Ca | Mg | Cu | Pb |
|---|---|---|---|---|---|---|---|---|---|---|---|---|---|
| 1. (BME-44) | ONPOE + PVC | – | -56.1 | 1-4 | -3.3 | -2.0 | -0.9 | -2.4 | -4.0 | -5.8 | -4.6 | -5.1 | -3.8 |
| | ONPOE + PVC | – | -55.2 | 1-5 | | | | | | | | | |
| | ONPOE + PVC | NaTPB | -59.3<br>-57.0<br>-55.6 | 1-4<br>1-5<br>1-6 | -3.5 | -2.2 | -0.9 | -2.4 | -4.3 | -6.1 | -4.9 | -5.2 | -4.0 |
| | DPP+PVC | | -58.9 | 1-4 | -3.4 | -2.1 | -0.9 | -2.4 | -3.7 | -5.0 | -4.7 | -4.7 | -4.6 |
| | | | -57.1 | 1-5 | | | | | | | | | |
| | SR | – | -58.2 | 1-4 | -3.4 | -1.9 | – | – | -4.1 | -4.4 | -4.5 | – | – |
| | | | -56.7 | 1-5 | | | | | | | | | |
| 2. (BME-53) | ONPOE + PVC | – | -55.6 | 1-4 | -3.3 | -2.1 | -0.9 | -2.3 | -3.5 | -4.1 | -4.3 | -3.9 | -3.6 |
| | | | -53.7 | 1-5 | | | | | | | | | |
| 3. (BME-54) | ONPOE + PVC | – | -55.9 | 1-4 | -3.4 | -2.1 | -0.9 | -2.3 | -3.5 | -4.7 | -4.5 | -4.2 | -4.3 |
| 4. (BME-36) | ONPOE + PVC | – | -40.4 | 1-4 | -2.2 | -1.5 | – | – | -3.2 | -3.3 | -3.4 | – | – |

Table 4 (continued)

| | | | | | | | | | | | | | |
|---|---|---|---|---|---|---|---|---|---|---|---|---|---|
| 5. (BME-15) | ONPOE + PVE | - | -54.0 | 1-5 | -3.2 | -2.0 | -1.0 | -2.1 | -3.9 | -3.6 | -4.6 | -3.8 | -3.5 |
| | ONPOE + PVC | NaTPB | -56.2 | 1-5 | -3.2 | -2.0 | -1.0 | -2.1 | -3.9 | -3.6 | -4.4 | -3.8 | -3.5 |
| | DPP + PVC | - | -57.1 | 1-4 | -3.0 | -2.0 | -1.0 | -2.3 | -3.6 | -4.7 | -4.5 | -3.8 | -3.5 |
| 6. (BME-15-Me) | ONPOE+PVC | - | -42.3 | 1-4 | -2.2 | -1.4 | - | - | -3.1 | -2.7 | -3.1 | - | - |
| 7. (BME-36/2) | ONPOE+PVC | - | -50.0 | 1-4 | -1.0 | -0.7 | - | - | -1.5 | -1.2 | -1.8 | - | - |
| 8. (BME-71) | ONPOE+PVC | - | -54.6 | 1-4 | -2.7 | -1.8 | - | - | - | -3.6 | -4.3 | - | - |
| 9.** ($R_V$) | | | -57.8 | 1-5 | -4.2 | -1.8 | +0.4 | -0.4 | -3.8 | -5.0 | -4.9 | -4.2 | -4.9 |
| 10.*** ($R_K$) | ONPOE+PVC | | -57.0 | 1-5 | -2.8 | -1.7 | -1.0 | - | - | -4.1 | -3.6 | -4.6 | - |

* To the calculation of the selectivity coefficient theoretical S-value (59.16 mV/decade) is used
** Philips IS 561 K (data measured by us)
*** data measured by us

Table 5

Selectivity characteristics of potassium ion-selective electrodes

| No. | Interfering cation j | Na | $NH_4$ | Rb | Cs | Li | Ca | Mg | Cu | Pb |
|---|---|---|---|---|---|---|---|---|---|---|
| | log $K_{ij}^{Pot}$ * | | | | | | | | | |
| 1 (BME-44) | | -3.5 | -2.2 | -0.9 | -2.4 | -4.3 | -6.1 | -4.9 | -5.2 | -4.0 |
| 3 (BME-54) | | -3.4 | -2.1 | -0.9 | -2.3 | -3.5 | -4.7 | -4.5 | -4.2 | -4.3 |
| 2 (BME-53) | | -3.1 | -2.1 | -0.9 | -2.3 | -3.5 | -4.1 | -4.3 | -3.9 | -3.6 |
| 5 (BME-15) | | -3.0 | -2.0 | -1.0 | -2.1 | -3.9 | -3.6 | -4.6 | -3.8 | -3.5 |
| 9 ($R_V$) ** | | -4.2 | -1.8 | +0.4 | -0.4 | -3.8 | -5.0 | -4.9 | -4.2 | -4.9 |
| 10 ($R_K$)*** | | -2.8 | -1.7 | -1.0 | - | - | -4.1 | -3.6 | -4.6 | - |

* To the calculation of the selectivity coefficient we used the theoretical S (59.16 mV(decade) value.

** Philips IS 561-K (data measured by us)

*** Data measured by us.

Table 6

Examination of the slope /S/ value of ion-selective electrodes

| No. | | | Condition in blood serum *** before S(mV/decade)** | after S(mV/decade)** |
|---|---|---|---|---|
| 1 | (BME-44) | a. | 57.5 | 57.9 |
| | | b. | 57.2 | 57.6 |
| 2 | (BME-53) | a. | 56.7 | 56.1 |
| | | b. | 57.2 | 56.3 |
| 3 | (BME-54) | a. | 56.8 | 57.2 |
| | | b. | 57.2 | 57.9 |
| 5 | (BME-15) | a. | 56.5 | 51.5 |

* Composition of electrode membrane: 4 mg ionophor
64 mg PVC
120 mg PNPOE
1 mg NaTPB

** Measured in $10^{-2}$ mol/l-$10^{-3}$ mol/ l KCl solution comprising 0.1 mol/l of NaCl

*** In the period between the measurements (1 month) the electrodes were continuously stored in blood serum (room temperature).

**Claims for the Contracting States: BE CH DE FR GB IT LI NL SE**

1. Crown compounds of general formula I

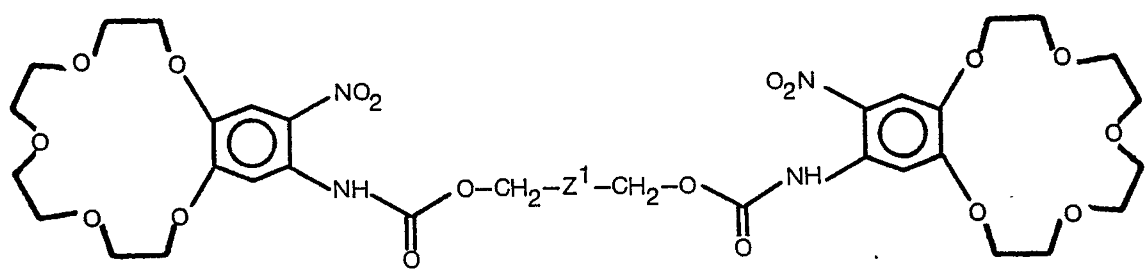

(I)

wherein

$Z^1$ is a group of formula

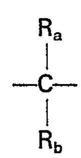

in which $R_a$ represents an alkyl group having 1—4 carbon atoms and $R_b$ represents an alkyl group having 1—20 carbon atoms.

2. A compound of general formula I according to claim 1 selected from
2,2-diethyl-propylene-1,3-bis-N,N′-[2″-nitro-4″,5″-(1‴,4‴,7‴,10‴,13‴-pentaoxacyclopentadeca-2‴-ene)-phenyl]-carbamate,
2,2-di-*n*-butyl-propylene-1,3-bis-N,N′-[2″-nitro-4″,5″-(1‴,4‴,7‴,10‴,13‴-pentaoxacyclopentadeca-2‴-ene)-phenyl]-carbamate,
2-*n*-dodecyl-2-methyl-propylene-1,3-bis-N,N′-[2″-nitro-4″,5″-(1‴,4‴,7‴,10‴,13‴-pentaoxacyclopentadeca-2‴-ene)-phenyl]-carbamate.

3. Process for the preparation of crown compounds of general formula I as defined in claim 1, which comprises

a) reacting a crown compound of formula II

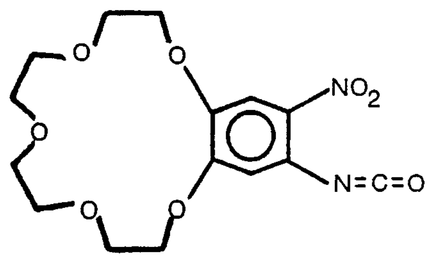

(II)

with a compound of formula III

$$HO—CH_2—Z^1—CH_2—OH$$

(wherein $Z^1$ is as defined in claim 1) in an aprotic solvent, or

b) reacting a crown compound of formula I′

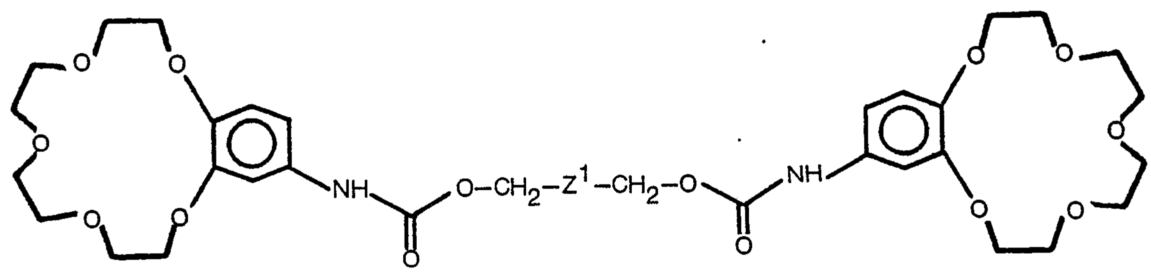

(I′)

(wherein $Z^1$ is as defined in claim 1) with nitric acid.

4. A potassium ion-selective membrane electrode which contains, as active substance at least one crown compound of general formula I as defined in claim 1.

5. An electrode according to claim 4 in which the membrane is formed from active substance, optionally mixed with a plasticizer and/or further additives, preferably sodium tetraphenyl borate or potassium p-chlorotetraphenyl borate, bound to a carrier, the compound(s) of formula I being present in an amount of 0.1—10% by weight of the membrane.

6. An electrode according to claim 5 in which the membrane comprises 0.2—10% by weight of active substance, 55—75% by weight of a plasticizer, said plasticizer being a substance having a dielectric constant of 2—30, and a carrier, said carrier being a polymer, preferably PVC or a silicon rubber, having a dielectric constant of 2—30.

7. An electrode according to claim 5 or claim 6 wherein the plasticizer comprises a phthalic acid ester, a sebacic acid ester or o-nitro-phenyl octyl ether.

8. An electrode according to any one of claims 4 to 7 wherein the membrane is bound to a substance which possesses electron and/or ion-conducting or semi-conducting properties, preferably to metallic silver, silver chloride wire, platinum wire, gold fibre, graphite rod or silica.

9. A membrane electrode according to any one of claims 4 to 8 wherein the active substance comprises at least one of the following:
2,2-diethyl-propylene-1,3-bis-N,N′-[2″-nitro-4″,5″-(1‴,4‴,7‴,10‴,13‴-pentaoxacyclopentadeca-2‴ene)-phenyl]-carbamate,
2,2-di-*n*-butyl-propylene-1,3-bis-N,N′-[2″-nitro-4″,5″-(1‴,4‴,7‴,10‴,13‴-pentaoxacyclopentadeca-2‴-ene)-phenyl]-carbamate,
2-*n*-dodecyl-2-methyl-propylene-1,3-bis-N,N′-[2″-nitro-4″,5″-(1‴,4‴,7‴,10‴,13‴-pentaoxacyclopentadeca-2‴ene)-phenyl]-carbamate.

**Claims for the Contracting State: AT**

1. A process for the preparation of crown compounds of general formula I

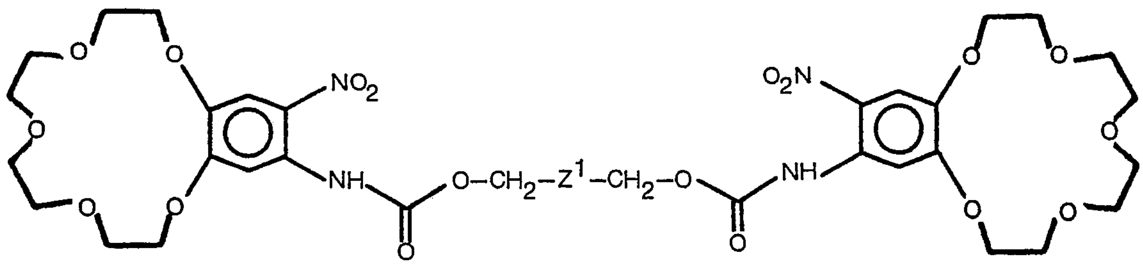

(I)

wherein

$Z^1$ is a group of formula

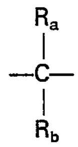

in which $R_a$ represents an alkyl group having 1—4 carbon atoms and $R_b$ represents an alkyl group having 1—20 carbon atoms, which comprises

a) reacting a crown compound of formula II

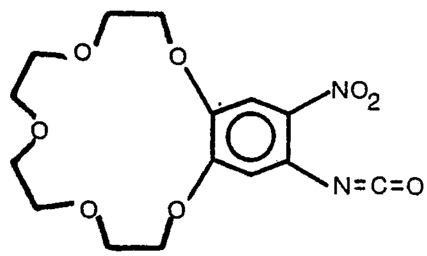

(II)

with a compound of formula III

$$HO—CH_2—Z^1—CH_2—OH \qquad (III)$$

(wherein $Z^1$ is as defined above) in an aprotic solvent, or

b) reacting a crown compound of formula I'

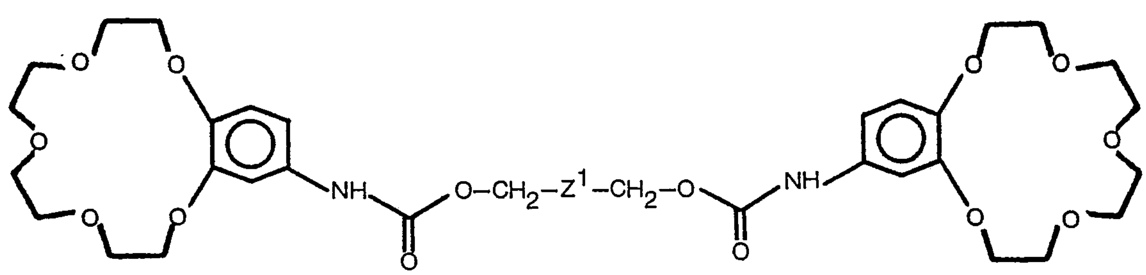

(I')

(wherein $Z^1$ is as defined above with nitric acid.

2. A process as claimed in claim 1 wherein $R_a$ and $R_b$ in the compound of formula III respectively each represent ethyl and ethyl, n-butyl and n-butyl, or methyl and n-dodecyl groups.

3. A process as claimed in claim 1 or claim 2 wherein reaction is carried out by reacting a compound of formula (II) with a compound of formula (III) in a molar ratio of 2:1 at a temperature of between 0° and 50°C in a chlorinated hydrocarbon or in an ether solvent.

4. A process as claimed in claim 1 or claim 2 wherein nitration of the compound of formula I' is carried out in an acetic acid or chlorinated hydrocarbon solvent at from 30° to 70°C.

5. The use, as active substance of at least one crown compound of general formula I

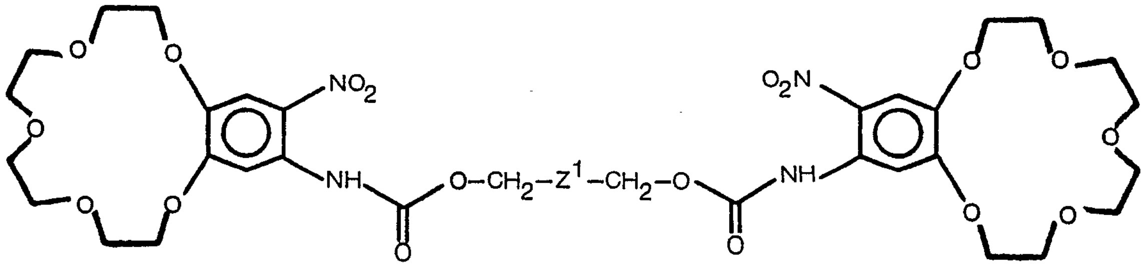

(I)

wherein

$Z^1$ is a group of formula

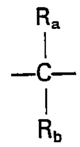

in which $R_a$ represents an alkyl group having 1—4 carbon atoms and $R_b$ represents an alkyl group having 1—20 carbon atoms in the preparation of a potassium ion-selective membrane electrode.

6. A method of producing a potassium ion-selective membrane electrode in which the membrane is formed from an active substance of formula (I) as defined in claim 5, optionally mixed with a plasticizer and/or further additives, preferably sodium tetraphenyl borate or potassium p-chlorotetraphenyl borate, bound to a carrier, the compound(s) of formula I being present in an amount of 0.1—10% by weight of the membrane.

7. A method as claimed in claim 6 in which the membrane is formed from 0.2—10% by weight of active substance, 55—75% by weight of a plasticizer, said plasticizer being a substance having a dielectric constant of 2—30, and a carrier, said carrier being a polymer, preferably PVC or a silicon rubber, having a dielectric constant of 2—30.

8. A method as claimed in claim 6 or claim 7 wherein the plasticizer comprises a phthalic acid ester, a sebacic acid ester or o-nitro-phenyl octyl ether.

9. A method as claimed in any of claims 6 to 8 wherein the membrane is bound to a substance which possesses electron and/or ion-conducting or semi-conducting properties, preferably to metallic silver, silver chloride wire, platinum wire, gold fibre, graphite rod or silica.

10. A method as claimed in any of claims 6 to 9 wherein the active substance comprises at least one of the following:

2,2-diethyl-propylene-1,3-bis-N,N′-[2″-nitro-4″,5″-(1‴,4‴,7‴,10‴,13‴-pentaoxacyclopentadeca-2‴ene)-phenyl]-carbamate,

2,2-di-*n*-butyl-propylene-1,3-bis-N,N′-[2″-nitro-4″,5″-(1‴,4‴,7‴,10‴,13‴-pentaoxacyclopentadeca-2‴-ene)-phenyl]-carbamate,

2-*n*-dodecyl-2-methyl-propylene-1,3-bis-N,N′-[2″-nitro-4″,5″-(1‴,4‴,7‴,10‴,13‴-pentaoxacyclopentadeca-2‴ene)-phenyl]-carbamate.

**Patentansprüche für die Vertragsstaaten: BE CH DE FR GB IT LI NL SE**

1. Kronenverbindungen der allgemeinen Formel I

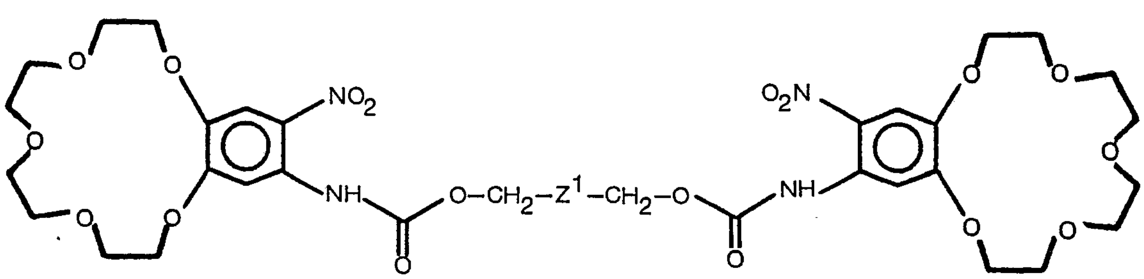

(I)

worin bedeuten:

$Z^1$ eine Gruppe der Formel

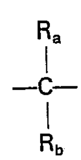

in welcher $R_a$ eine Alkylgruppe mit 1—4 Kohlenstoffatomen ist und $R_b$ eine Alkylgruppe mit 1—20 Kohlenstoffatomen ist.

2. Verbindung der allgemeinen Formel I gemäß Anspruch 1 ausgewählt aus.

2,2-Diethyl-propylen-1,3-bis-N,N'-[2''-nitro-4'',5''-(1''',4''',7''',10''',13'''-pentaoxacyclopentadeca-2'''-en)-phenyl]-carbamat,

2,2-Di-*n*-butyl-propylen-1,3-bis-N,N'-[2''-nitro-4'',5''-(1''',4''',7''',10''',13'''-pentaoxacyclopentadeca-2'''-en)-phenyl]-carbamat,

2-*n*-Dodecyl-2-methyl-propylen-1,3-bis-N,N'-[2''-nitro-4'',5''-(1''',4''',7''',10''',13'''-pentaoxacyclopentadeca-2'''en)-phenyl]-carbamat.

3. Verfahren zur Herstellung von Kronenverbindungen der allgemeinen Formel I gemäß Anspruch 1, dadurch gekennzeichnet, daß man

a) eine Kronenverbindung der Formel II

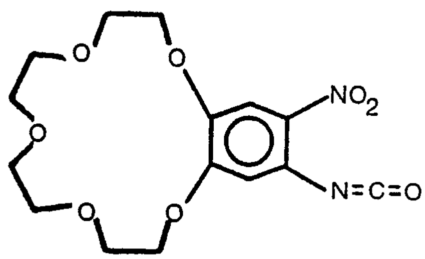

(II)

mit einer Verbindung der Formel III

$$HO—CH_2—Z^1—CH_2—OH \quad (III)$$

(worin $Z^1$ die in Anspruch 1 angegebene Bedeutung hat) in einem aprotischen Lösungsmittel umsetzt, oder

b) eine Kronenverbindung der formel I'

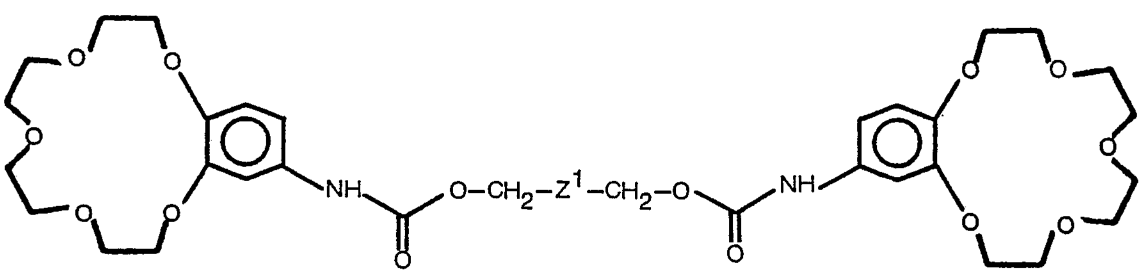

(I')

(worin $Z^1$ die in Anspruch 1 angegebene Bedeutung hat) mit Salpetersäure umsetzt.

4. Kaliumionenselektive Membranelektrode, enthaltend als aktive Substanz wenigstens eine Kronenverbindung der allgemeinen Formel I gemäß Anspruch 1.

5. Elektrode gemäß Anspruch 4, in welcher die Membran aus einer aktiven Substanz, gewünschtenfalls mit einem Weichmacher und/oder weiteren Additiven, vorzugsweise Natriumtetraphenylborat oder Kaliumparachlortetraphenylborat ausgebildet ist, gebunden an einen Träger, wobei die Verbindung(en) der Formel I in einer Menge von 0,1—10 Gew.-% der Membran vorhanden ist bzw. sind.

6. Elektrode gemäß Anspruch 5, in welcher die Membran 0,2—10 Gew.-% der aktiven Substanz, 55—75 Gew.-% eines Weichmachers umfaßt, wobei der Weichmacher eine Substanz mit einer Dielektrizitätskonstante von 2—30 ist, sowie einen Träger, wobei der Träger ein Polymer, vorzugsweise PVC, oder ein Silikonkautschuk mit einer Dielektrizitätskonstante von 2—30 ist.

7. Elektrode gemäß Anspruch 5 oder Anspruch 6, in welcher der Weichmacher einen Phthalsäureester, einen Sebacinsäureester oder ortho-Nitrophenyloctylether umfaßt.

8. Elektrode gemäß einem der Ansprüche 4 bis 7, in welcher die Membran an eine Substanz, die Elektronen und/oder ionenleitende oder halbleitende Eigenschaften aufweist, vorzugsweise an metallisches Silber, Silberchloriddraht, Platindraht, Goldfaser, einen Graphitstab oder Siliziumdioxid gebunden ist.

9. Membranelektrode gemäß einem der Ansprüche 4 bis 8, in welcher die aktive Substanz wenigstens eine der folgenden Verbindungen umfaßt:

2,2-Diethyl-propylen-1,3-bis-N,N'-[2''-nitro-4'',5''-(1''',4''',7''',10''',13'''-pentaoxacyclopentadeca-2'''-en)-phenyl]-carbamat,

2,2-Di-*n*-butyl-propylen-1,3-bis-N,N'-[2''-nitro-4'',5''-(1''',4''',7''',10''',13'''-pentaoxacyclopentadeca-2'''-en)-phenyl]-carbamat,

2-*n*-Dodecyl-2-methyl-propylen-1,3-bis-N,N'-[2''-nitro-4'',5''-(1''',4''',7''',10''',13'''-pentaoxacyclopentadeca-2'''en)-phenyl]-carbamat.

**Patentansprüche für den Vertragsstaat: AT**

1. Verfahren zur Herstellung von Kronenverbindungen der allgemeinen Formel I

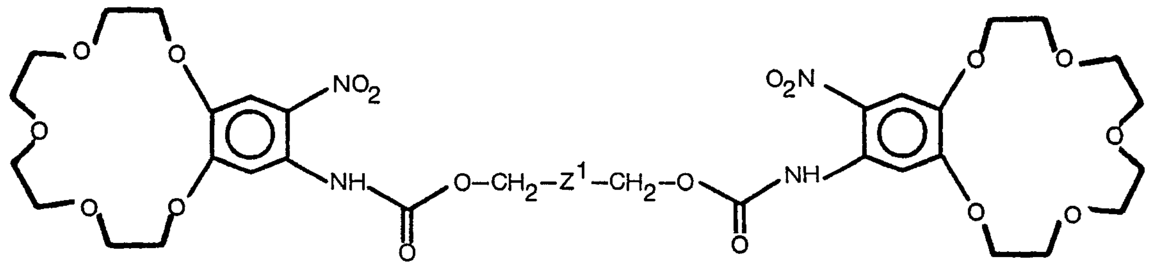

(I)

in welcher $Z^1$ eine Gruppe der Formel

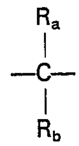

bedeutet, worin $R_a$ eine Alkylgruppe mit 1—4 Kohlenstoffatomen und $R_b$ eine Alkylgruppe mit 1—20 Kohlenstoffatomen ist, dadurch gekennzeichnet, daß man

a) eine Kronenverbindung der Formel II

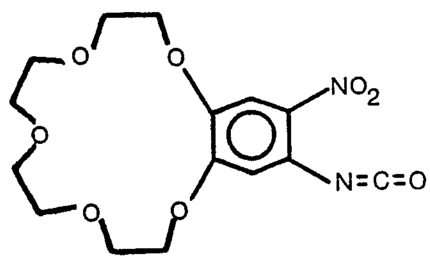

(II)

mit einer Verbindung der Formel III

$$HO—CH_2—Z^1—CH_2—OH \qquad (III)$$

(worin $Z^1$ die früher angegebene Bedeutung hat) in einem aprotischen Lösungsmittel umsetzt, oder

b) eine Kronenverbindung der Formel I'

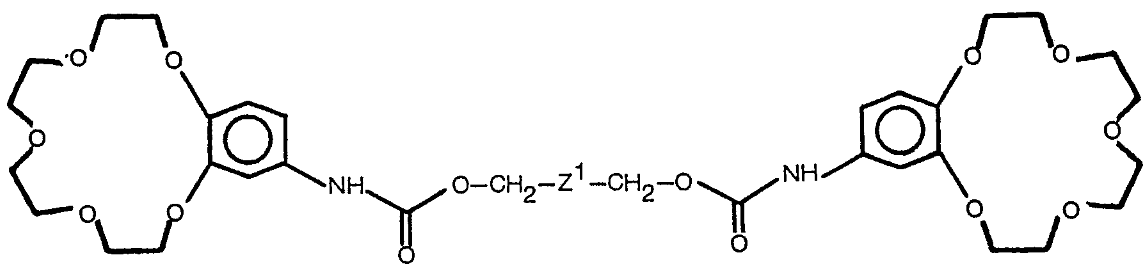

(I')

(in welcher $Z^1$ die vorher angegebene Bedeutung hat) mit Salpetersäure umsetzt.

2. Verfahren gemäß Anspruch 1, in welcher $R_a$ und $R_b$ in der Verbindung der Formel III jeweils Ethyl- und Ethyl-, n-Butyl- und N-Butyl-, oder Methyl- und N-Dodecylgruppen bedeuten.

3. Verfahren gemäß Anspruch 1 oder 2, bei welchem die Umsetzung durchgeführt wird, indem man eine Verbindung der Formel II mit einer Verbindung der Formel II in einem Molverhältnis von 2:1 bei einer Temperatur zwischen 0° und 50°C in einem chlorierten Kohlenwasserstoff oder in einem Ether als Lösungsmittel durchführt.

4. Verfahren gemäß Anspruch 1 oder Anspruch 2, bei welcher man die Nitrierung der Verbindung der Formel I' in Essigsäureanhydrid oder einem chlorierten Kohlenwasserstofflösungsmittel bei 30° bis 70°C durchführt.

5. Verwendung als aktive Substanz wenigstens einer Kronenverbindung der allgemeinen Formel I

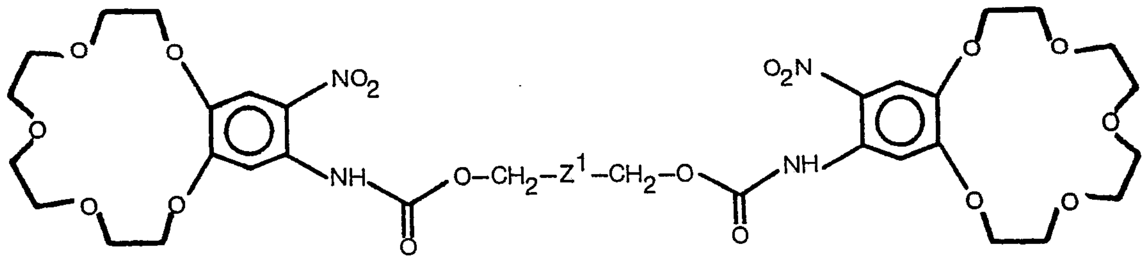 (I)

worin $Z^1$ eine Gruppe der Formel

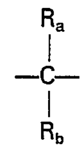

ist, in welcher $R_a$ eine Alkylgruppe mit 1—4 Kohlenstoffatomen und $R_b$ eine Alkylgruppe mit 1—20 Kohlenstoffatomen ist, bei der Herstellung einer kaliumionenselektiven Membranelektrode.

6. Verfahren zur Herstellung einer kaliumionenselektiven Membranelektrode, bei welcher die Membran aus einer aktiven Substanz der Formel I gemäß der Definition in Anspruch 5, gewünschtenfalls in Abmischung mit einem Weichmacher und/oder weiteren Additiven, vorzugsweise Natriumtetraphenylborat oder Kaliumparachlortetraphenylborat, gebunden an einen Träger, ausgebildet ist, wobei die Verbindung(en) der Formel I in einer Menge von 0,1—10 Gew.-% der Membran vorhanden ist bzw. sind.

7. Verfahren gemäß Anspruch 6, bei welchem die Membran aus 0,2—10 Gew.-% aktiver Substanz, 55—75 Gew.-% Weichmacher, wobei der Weichmacher eine Substanz ist mit einer Dielektrizitätskonstante von 2—30, und einem Träger ausgebildet ist, wobei der Träger ein Polymer ist, vorzugsweise PVC oder ein Silikonkautschuk, mit einer Dielektrizitätskonstante von 2—30.

8. Verfahren gemäß Anspruch 6 oder Anspruch 7, bei welchem der Weichmacher ein Phthalsäureester, einen Sebacinsäureester oder ortho-Nitrophenyloctylether umfaßt.

9. Verfahren gemäß einem der Ansprüche 6 bis 8, bei welchem die Membran an ein Substrat gebunden ist, welches Elektronen und/oder ionenleitende oder halbleitende Eigenschaften aufweist, vorzugsweise an metallisches Silber, Silberchloriddraht, Platindraht, Goldfaser, einen Graphitstab oder Siliziumdioxid gebunden ist.

10. Verfahren gemäß einem der Ansprüche 6 bis 9, bei welchem die aktive Substanz wenigstens einer folgenden Verbindungen umfaßt:
2,2-Diethyl-propylen-1,3-bis-N,N'-[2''-nitro-4'',5''-(1''',4''',7''',10''',13'''-pentaoxacyclopentadeca-2'''-en)-phenyl]-carbamat,
2,2-Di-*n*-butyl-propylen-1,3-bis-N,N'-[2''-nitro-4'',5''-(1''',4''',7''',10''',13'''-pentaoxacyclopentadeca-2'''-en)-phenyl]-carbamat,
2-*n*-Dodecyl-2-methyl-propylen-1,3-bis-N,N'-[2''-nitro-4'',5''-(1''',4''',7''',10''',13'''-pentaoxacyclopentadeca-2'''en)-phenyl]-carbamat.

**Revendications pour les Etats contractants: BE CH DE FR GB IT LI NL SE**

1. Composés en couronne de formule générale I

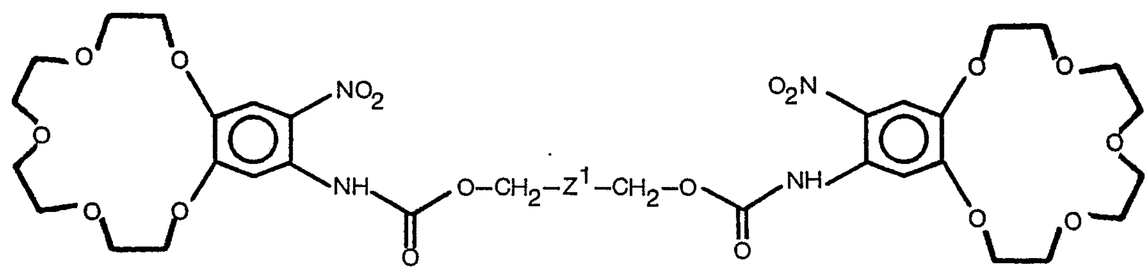 (I)

dans laquelle $Z^1$ est un groupe de formule

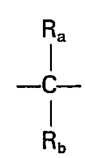

dans laquelle $R_a$ représente un groupe alkyle ayant 1 à 4 atomes de carbone et $R_b$ représente un groupe alkyle ayant 1 à 20 atomes de carbone.

2. Composé de formule générale I, selon la revendication 1, choisi parmi:
le 2,2-diéthyl-propylène-1,3-bis-N,N'-(2''-nitro-4'',5''-(1''',4''',7''',10''',13'''-pentaoxacyclopentadéca-2'''-ène)-phényl)carbamate,
le 2,2-diéthyl-propylène-1,3-bis-N,N'-(2''-nitro-4'',5''-(1''',4''',7''',10''',13'''-pentaoxacyclopentadéca-2'''-ène)-phényl)carbamate,
le 2,2-di-*n*-butyl-propylène-1,3-bis-N,N'-(2''-nitro-4'',5''-(1''',4''',7''',10''',13'''-pentaoxacyclopentadéca-2'''-ène)-phényl)-carbamate,
le 2-*n*-dodécyl-2-méthyl-propylène-1,3-bis-N,N'-(2''-nitro-4'',5''-(1''',4''',7''',10''',13'''-pentaoxacyclopentadéca-2'''-ène)-phényl)-carbamate.

(a) la réaction d'un composé en couronne de formule II

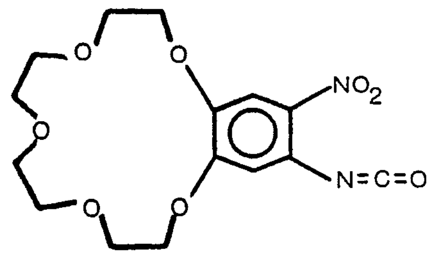

(II)

avec un composé de formule III

$$HO—CH_2—Z^1—CH_2—OH \quad (III)$$

(dans laquelle $Z^1$ est tel que défini dans la revendication 1) dans un solvant aprotique, ou

b) la réaction d'un composé en couronne de formule I'

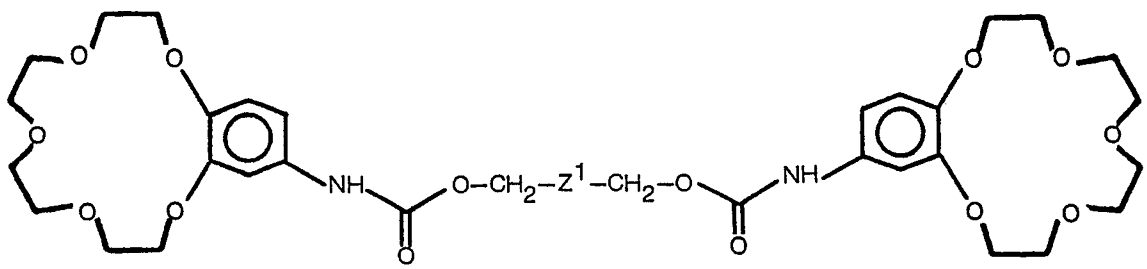

(I')

(dans laquelle $Z^1$ est tel que défini dans la revendication 1) avec l'acide nitrique.

4. Electrode à membrane sélective de l'ion potassium qui contient, à titre de substance active, au moins un composé en couronne de formule générale I tel que défini dans la revendication 1.

5. Electrode selon la revendication 4, dans laquelle la membrane est formée à partir de la substance active, éventuellement en mélange avec un plastifiant et/ou d'autres additifs, de préférence le tétraphénylborate de sodium ou le p-chlorotétraphénylborate de potassium, liée à un support, le(s) composé(s) de formule I étant présent(s) en une quantité comprise entre 0,1 et 10% en poids de la membrane.

6. Electrode selon la revendication 5, dans laquelle la membrane comprend 0,2 à 10% en poids de substance active, 55 à 75% en poids d'un plastifiant, ledit plastifiant étant une substance ayant une constante diélectrique comprise entre 2 et 30, et un support, ledit support étant un polymère, de préférence du PVC ou un caoutchouc au silicium, ayant une constante diélectrique comprise entre 2 et 30.

7. Electrode selon la revendication 5 ou 6, dans laquelle le plastifiant englobe un ester de l'acide phtalique, un ester de l'acide sébacique ou un o-nitro-phényl-octyl-éther.

8. Electrode selon l'une quelconque des revendications 4 à 7, dans laquelle la membrane est liée à une substance qui possède des propriétés électroniques et/ou conductrices d'ions ou semi-conductrices, de préférence de l'argent métallique, un fil de chlorure d'argent, un fil de platine, une fibre d'or, une tige de graphite ou de la silice.

9. Electrode à membrane selon l'une quelconque des revendications 4 à 8, dans laquelle la substance active engloble au moins l'une des substances suivantes:
le 2,2-diethyl-propylène-1,3-bis-N,N'-[2''-nitro-4'',5''-(1''',4''',7''',10''',13'''-pentaoxacyclopentadéca-2'''-ène)-phényl)-carbamaté,
le 2,2-di-*n*-butyl-propylène-1,3-bis-N,N'-[2''-nitro-4'',5''-(1''',4''',7''',10''',13'''-pentaoxacyclopentadéca-2'''-ène)-phényl)-carbamate,
le 2-*n*-dodecyl-2-méthyl-propylène-1,3-bis-N,N'-[2''-nitro-4'',5''-(1''',4''',7''',10''',13'''-pentaoxacyclopentadéca-2'''ène)-phényl)-carbamate.

**Revendications pour l'etat contractant: AT**

1. Procédé de préparation de composés en couronne de formule générale I

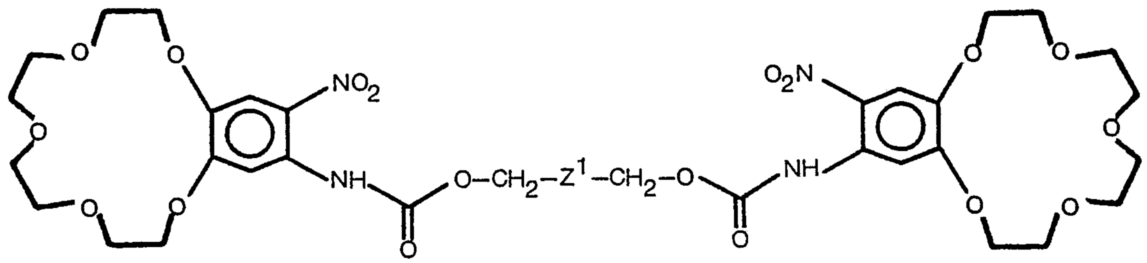

(I)

dans laquelle $Z^1$ est un groupe de formule

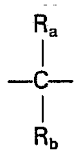

dans laquelle $R_a$ représente un groupe alkyle ayant 1 à 4 atomes de carbone et $R_b$ représente un groupe alkyle ayant 1 à 20 atomes de carbone, qui comprend

a) la réaction d'un composé en couronne de formule II

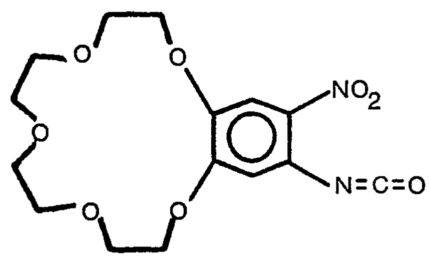

(II)

avec un composé en couronne de formule III

$$HO—CH_2—Z^1—CH_2—OH \qquad (III)$$

(dans laquelle $Z^1$ est tel que défini ci-dessus), dans un solvant aprotique, ou

b) la réaction d'un composé en couronne de formule I'

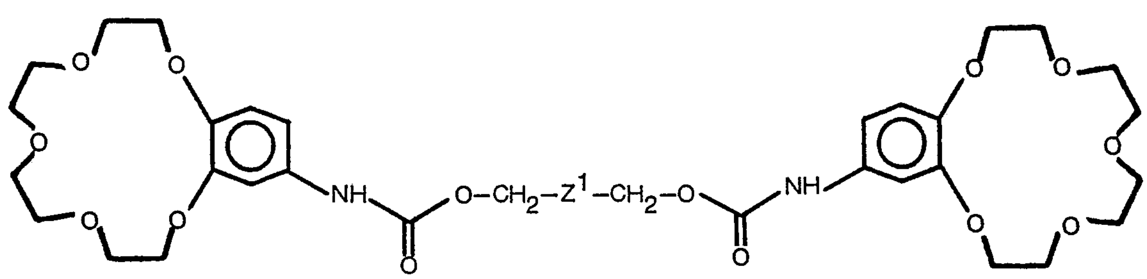

(I')

(dans laquelle $Z^1$ est tel que défini ci-dessus) avec l'acide nitrique.

2. Procédé selon la revendication 1, dans lequel $R_a$ et $R_b$, dans le composé de formule III, représentent chacun respectivement les groupes éthyle et éthyle, n-butyle et n-butyle, ou méthyle et n-dodécyle.

3. Procédé selon la revendication 1 ou 2, dans lequel on effectue la réaction en faisant réagir un composé de formule (II) avec un composé de formule (III), dans un rapport molaire de 2:1, à une température comprise entre 0° et 50°C, dans un hydrocarbure chloré ou dans un solvant éther.

4. Procédé selon la revendication 1 ou 2, dans lequel la nitration du composé de formule I' est réalisée dans un solvant acide acétique ou hydrocarbure chloré, à une température de 30° à 70°C.

5. Utilisation, à titre de substance active, d'au moins un composé en couronne de formule générale I

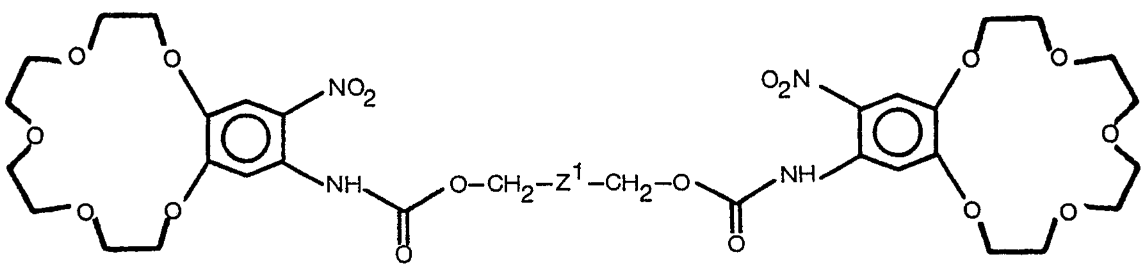 (I)

dans laquelle $Z^1$ est un groupe de formule

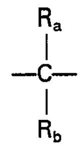

dans laquelle $R_a$ représente un groupe alkyle ayant 1 à 4 atomes de carbone et $R_b$ représente un groupe alkyle ayant 1 à 20 atomes de carbone, dans la préparation d'une électrode à membrane sélective de l'ion potassium.

6. Procédé de production d'une électrode à membrane sélective de l'ion potassium, dans lequel la membrane est formée à partir d'une substance active de formule (I), telle que définie dans la revendication 5, éventuellement en mélange avec un plastifiant et/ou d'autres additifs, de préférence le tétraphénylborate de sodium ou le p-chlorotétraphenylborate de potassium, lié à un support, le(s) composé(s) de formule I étant présent(s) en une quantité comprise entre 0,1 et 10% en poids de la membrane.

7. Procédé selon la revendication 6, dans lequel la membrane est formée à partir de 0,2 à 10% en poids de substance active, de 55 à 75% en poids d'un plastifiant, ledit plastifiant étant une substance ayant une constante diélectrique comprise entre 2 et 30, et d'un support, ledit support étant un polymère, de préférence du PVC ou un caoutchouc au silicium, ayant une constance diélectrique comprise entre 2 et 30.

8. Procédé selon la revendication 6 ou 7, dans lequel le plastifiant englobe un ester de l'acide phtalique, un ester de l'acide sébacique ou un o-nitro-phényl-octyl-éther.

9. Procédé selon l'une quelconque des revendications 6 à 8, dans lequel le membrane est liée à une substance qui possède des propriétés électroniques et/ou conductrices d'ions ou semi-conductrices, de préférence de l'argent métallique, un fil de chlorure d'argent, un fil de platine, une fibre d'or, une tige de graphite ou de la silice.

10. Procédé selon l'une quelconque des revendications 6 à 9, dans lequel la substance active englobe au moins l'une des substances suivantes:
le 2,2-diethyl-propylène-1,3-bis-N,N'-[2''-nitro-4'',5''-(1''',4''',7''',10''',13'''-pentaoxacyclopentadéca-2'''-ène)-phényl]-carbamaté,
le 2,2-di-*n*-butyl-propylène-1,3-bis-N,N'-[2''-nitro-4'',5''-(1''',4''',7''',10''',13'''-pentaoxacyclopentadéca-2'''-ène)-phényl]-carbamate,
le 2-*n*-dodecyl-2-méthyl-propylène-1,3-bis-N,N'-[2''-nitro-4'',5''-(1''',4''',7''',10''',13'''-pentaoxacyclopentadéca-2'''ène)-phényl]-carbamate.

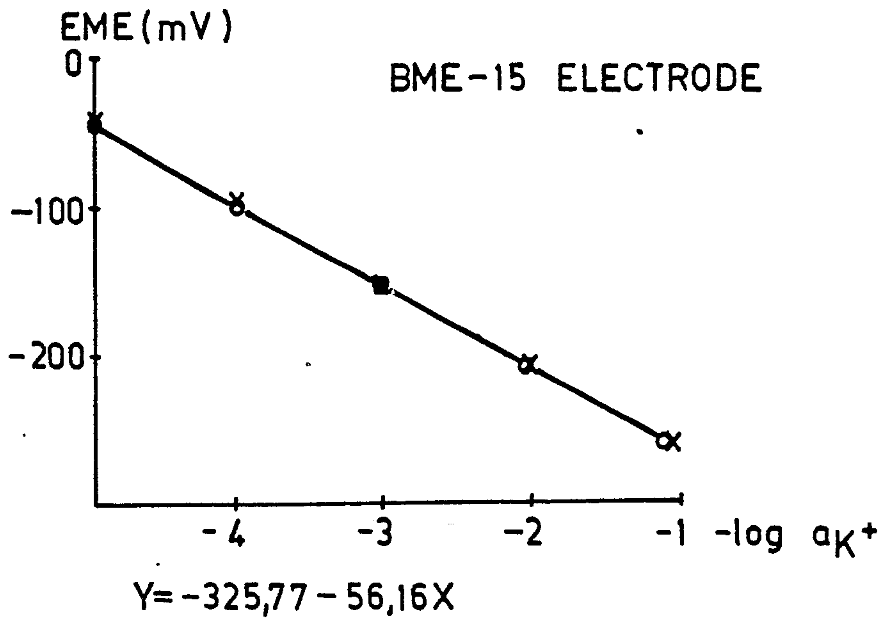


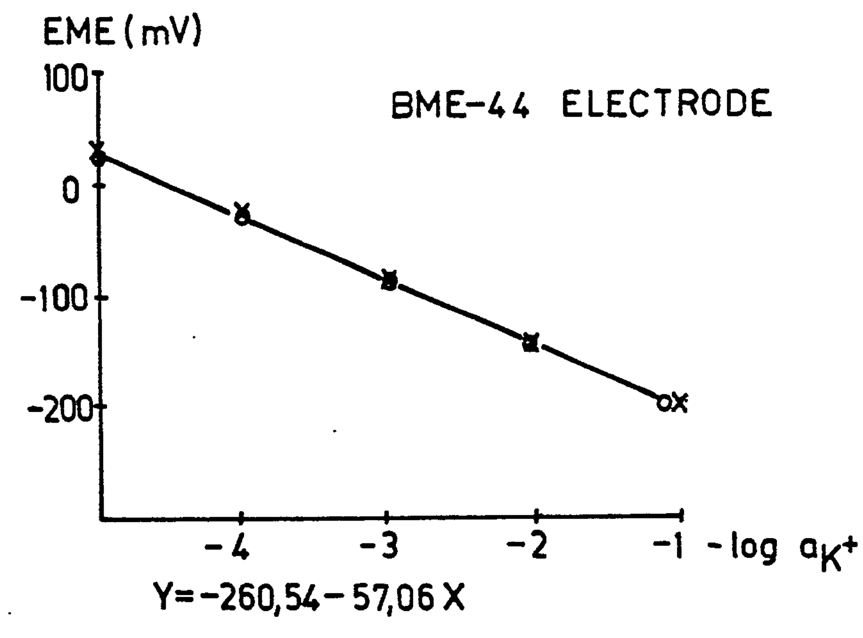


Fig. 1

Calibration data of potassium ion selective electrodes

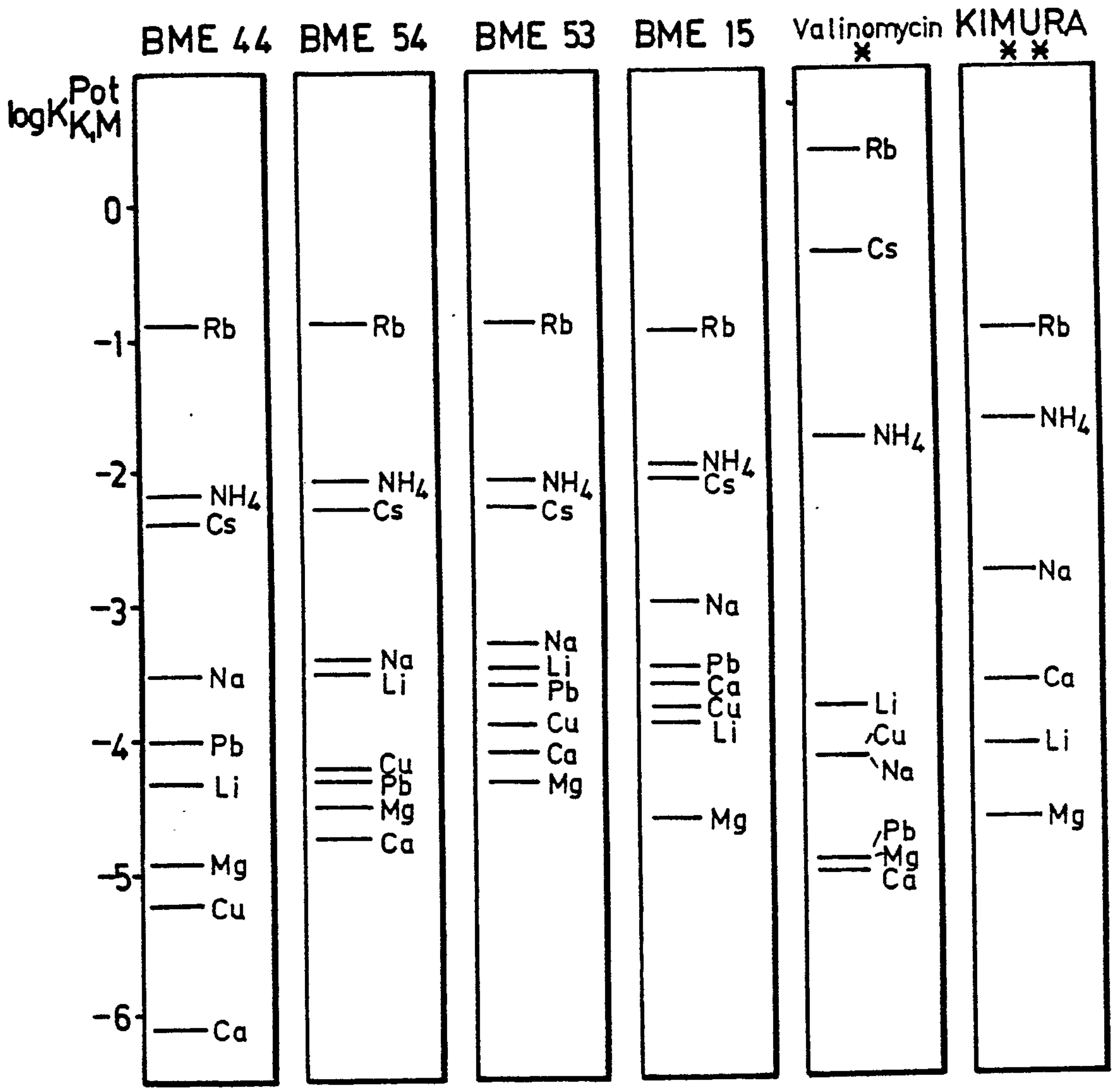


* Philips IS 561-K+

** KIMURA,K., MAEDA,T., TAMURA,H., SHONO,T., J. Electroanal. Chem., 95 /1979/ 91-101

Fig. 2

Comparison of potassium ion selective electrodes

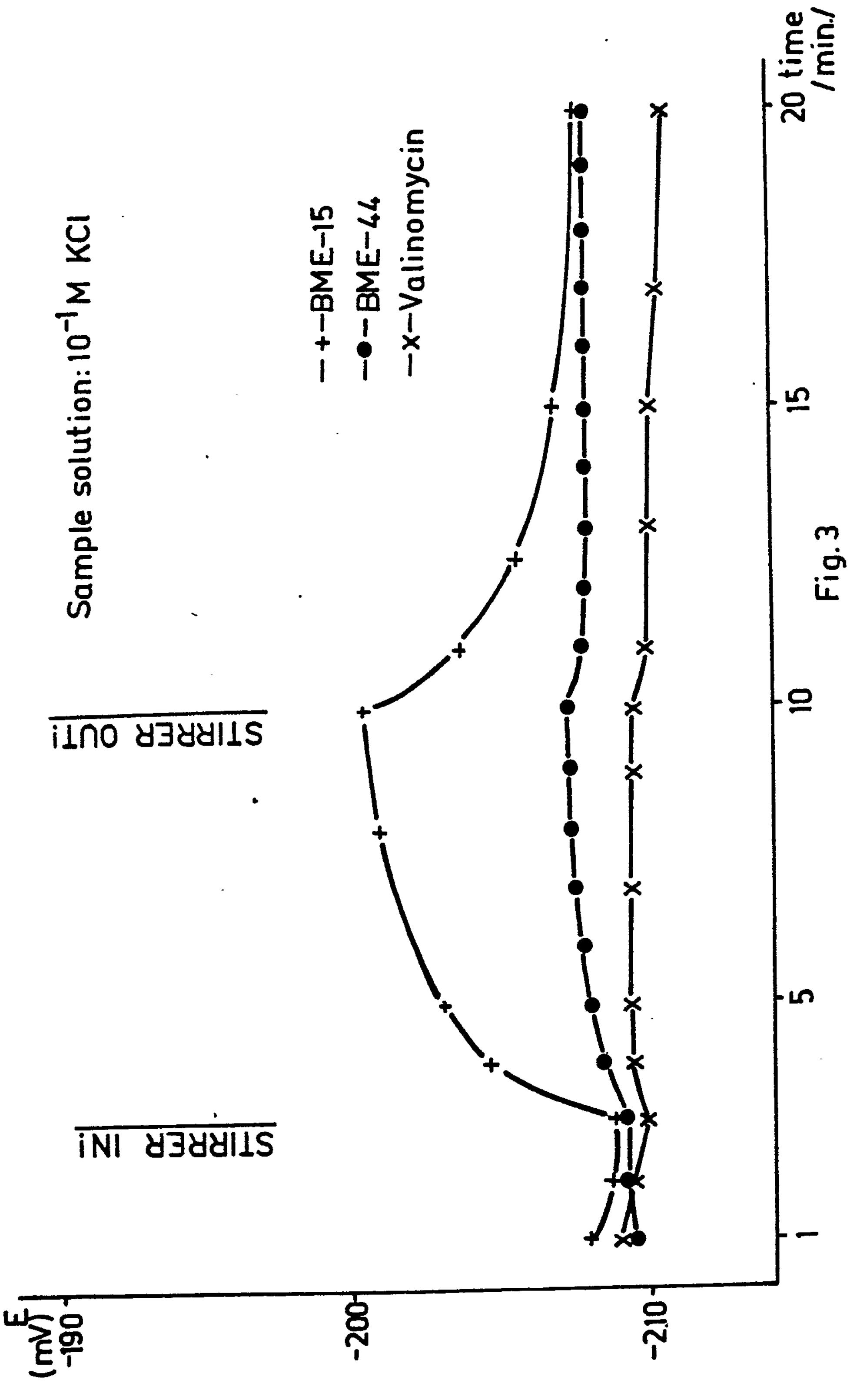


Fig. 3

Dependence of the sign measured by potassium ion-selective electrodes on stirring rate